(19) Europäisches Patentamt
European Patent Office
Office européen des brevets

(11) Veröffentlichungsnummer : **0 419 980 B1**

(12) # EUROPÄISCHE PATENTSCHRIFT

(45) Veröffentlichungstag der Patentschrift :
**18.08.93 Patentblatt 93/33**

(51) Int. Cl.$^5$ : **C07C 255/07,** C07C 253/30, C11B 9/00

(21) Anmeldenummer : **90117788.1**

(22) Anmeldetag : **15.09.90**

(54) **Alkadiennitrile, Verfahren zu ihrer Herstellung und ihre Verwendung.**

(30) Priorität : **28.09.89 DE 3932325**

(43) Veröffentlichungstag der Anmeldung :
**03.04.91 Patentblatt 91/14**

(45) Bekanntmachung des Hinweises auf die Patenterteilung :
**18.08.93 Patentblatt 93/33**

(84) Benannte Vertragsstaaten :
**CH DE FR GB IT LI NL**

(56) Entgegenhaltungen :
**EP-A- 0 017 396**
**GB-A- 686 692**
**US-A- 3 531 510**
**US-A- 3 655 722**

(73) Patentinhaber : **HAARMANN & REIMER GMBH**
**Postfach 12 53**
**D-37601 Holzminden (DE)**

(72) Erfinder : **Hopp, Rudolf, Dr.**
**Auf dem Gehrenkamp 28**
**W-3450 Holzminden (DE)**
Erfinder : **Thielmann, Thomas, Dr.**
**Bruder-Grimm-Weg 19**
**W-3450 Holzminden (DE)**
Erfinder : **Göttsch, Wilhelm**
**Eichenhang 12**
**W-3454 Bevern (DE)**

(74) Vertreter : **Müller, Gerhard, Dr. et al**
**BAYER AG Konzernverwaltung RP Patente Konzern**
**D-51368 Leverkusen (DE)**

EP 0 419 980 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Alkadiennitrile in Verfahren zu ihrer Herstellung und ihre Verwendung als Riechstoffe.

Es wurden neue Alkadiennitrile der Formel

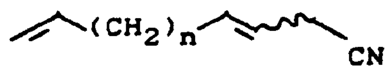

(I)

in der n eine ganze Zahl von 5 bis 9, vorzugsweise 7 bis 9, ist, gefunden, die wertvolle sensorische Eigenschaften aufweisen.

Die erfindungsgemäßen Alkadiennitrile der Formel (I) werden durch Kondensation von Alkenalen der Formel

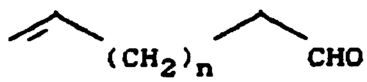

(II),

in der n die unter Formel (I) angegebene Bedeutung hat, mit Cyanessigsäure erhalten.

Die Erfindung betrifft daher auch ein Verfahren zur Herstellung von Alkadiennitrilen der Formel (I), das dadurch gekennzeichnet ist, daß man Alkenale der Formel (II) mit Cyanessigsäure in Gegenwart von N,N-Dimethylbenzylamin, in einem inerten Lösungsmittel, wie aromatischen Kohlenwasserstoffen, vorzugsweise Toluol, bei Rückflußtemperatur kondensiert. Das bei der Reaktion als Nebenprodukt in geringen Mengen ($<$ 20 Gew.-%) entstehende $\alpha,\omega$-Alkadiennitril (Cis-trans-Isomerengemisch) muß nicht abgetrennt werden, da es die Geruchseigenschaften der erfindungsgemäßen $\beta,\omega$-Alkadiennitrile der Formel (I) nicht beeinträchtigt. Die erfindungsgemäßen $\beta,\omega$-Alkandiennitrile fallen in Form eines Gemisches der Cis- und trans-Isomere an Das Gewichtsverhältnis von Cis- und Trans-Isomeren schwankt in Abhängigkeit von den Reaktionsbedingungen zwischen 1:5 bis 1:1. Cis- und Trans-Isomere unterscheiden sich zwar etwas in ihren Geruchsnoten, eine Trennung ist jedoch für ihre Verwendung als Riechstoff nicht erforderlich.

Die als Ausgangsstoffe benötigten Alkenale der Formel (II) sind Handelsprodukte oder nach bekannten Verfahren herstellbar (siehe A.J. A. van der Weerdt et. al. in "Progress in Essential Oil Research" W.d. Gryter, Berlin 1986, S. 215).

Die erfindungsgemäßen neuen Alkadiennitrile der Formel (I) sind Riechstoffe, die einerseits die Stabilität und die gute Haftung der bekannten Nitrile wie z.B. des Tridecennitrils (siehe GB-PS 686 692) aufweisen, sich aber andererseits von diesen durch eine günstigere Geruchsnote, nämlich eine wesentlich natürlichere Note, unterscheiden. Überraschenderweise fehlt ihnen die unangenehme metallische Nebennote der bekannten Nitrile.

So lauten einige beispielsmäßige Geruchsbeschreibungen:

3,12-Tridecadiennitril (Isomerengemisch):

frisch citrisch-mandarinig, natürlich, leicht fruchtigt, sehr duftstark;

cis-3,12-Tridecadiennitril, intensive Mandarinennote, trans-3,12-Tridecadiennitril, weniger intensive Mandarinennote, aber weicher blumiger, etwas fettig;

3,13-Tetradecadiennitril (Isomerengemisch):

sehr duftstark, fruchtig, wachsig, nussig mit einer Note überreifer Mandarinen oder Orangen, guthaftend.

US-A-3 655 722 beschreibt Diennitrile, die nach Zitronen riechen.

Die erfindungsgemäßen Riechstoffe werden in Kombination mit anderen, an sich bekannten Riechstoffen (arctander, Perfume and Flavor Chemicals, Montclair, N. J. (USA), 1969) und etherischen Ölen (arctander, Perfume and Flavor Materials of natual Origin, Elisabeth, J.J. (USA), 1960) angewendet und führen zu Parfümbasen und Riechstoffkompositionen mit ausdruckstarken Noten, die sich hervorragend für die Parfümierung von Fertigprodukten des Aerosol-, Waschmittel- und des Chemisch-Technischen-Sektors, insbesondere aber des Feinparfümerie- und Kosmetiksektors eignen, z.B. für Detergentien, Haarpflegemittel, Schaumbäder, Badesalz, Geschirrspülmittel Waschpulver, Seifen, Antiperspirans, Puder, Cremes, Rasierwasser, After-Shave-Lotions, Raumluftverbessererr, WC-Reiniger, Raum-Sprays, Antiperspirans-Sprays, Deodorant-Sprays, Körper-Sprays, Sonnenschutzmittel.

In diesen Präparaten werden die erfindungsgemäßen Riechstoffe im allgemeinen in einer Menge von 0.001 bis 1 Gew-% vorzugsweise von 0.01 bis 0.5 Gew.-%, bezogen auf das fertige Präparat, eingesetzt.

Die Herstellung der Parfümkompositionen und parfümierten Produkte erfolgt in üblicher Weise, beispielsweise durch Zusammengeben der Komponenten.

Beispiel 1

In einem Dreihalskolben, der mit Rührer, Wasserabscheider und Gaszähluhr ausgestattet ist, werden 168 g (1 Mol) 10-Undecenal, 135 g (1 Mol) N.N-Dimethylbenzylamin, 93 g (1,1 Mol) Cyanessigsäure und 20 g Wasser in 300 ml Toluol unter Wasserabscheidung auf Rückflußtemperatur erhitzt. Innerhalb von 60 Minuten werden ca. 25 l $CO_2$ und 37 g Wasser abgeschieden. Das abgekühlte Reaktionsgemisch wird nacheinander mit 250 g 10%iger Schwefelsäure und 250 g 5%iger Natriumhydrogencarbonatlösung gewaschen. Der nach dem Entfernen des Toluols verbleibende Rückstand wird bei 107 bis 135°C und 1 mbar destilliert. Man erhält 113 g 3,12-Tridecadiennitril (Isomerengemisch) mit einem Siedebereich von 114-116°C (2 mbar).

Durch Ersatz des 10-Undecenals durch eine äquivalente Menge 9-Decenal oder 11-Dodecenal werden 3,11- Dodecadiennitril (Isomerengemisch) (Sdp.: 90°C/1 mbar) bzw. 3,13-Tetradecadiennitril (Isomerengemisch) erhalten.

Beispiel 2

Eine Riechstoffkomposition wird durch Mischen folgender Bestandteile hergestellt (Mengenangabe in Gramm):

| | |
|---|---|
| n-Decanal | 20,0 |
| Trimethylundecylenaldehyd | 2,0 |
| Decylacetat | 100,0 |
| cis-3-Hexenol | 2,0 |
| Isotridecylacetat | 80,0 |
| Linalylacetat | 50,0 |
| Terpinylacetat | 50,0 |
| Citral | 2,0 |
| 3,12-Tridecadiennitril (Isomerengemisch) | 2,0 |
| Orangenöl (brasilian.) | 400,0 |
| β-Methylnaphthylketon | 50,0 |
| Limonen | 200,0 |
| Ethylbutyrat | 2,0 |
| Diethylphthalat | 48,0 |
| Jonol | 2,0 |
| | 1010,0 |

Durch den Zusatz von 3,12-Tridecadiennitril (Isomerengemisch) erhält die Komposition eine größere Duftfülle und insbesondere eine größere Natürlichkeit.

Beispiel 3

Eine Riechstoffkomposition wird durch Mischen folgender Bestandteile hergestellt (Mengenangaben in Gramm):

| | |
|---|---|
| 10-Undecenal | 2,0 |
| Galbanum Resin | 10,0 |
| Linalylacetat | 90,0 |
| Dihydromyrcenol | 200,0 |
| 3,12-Tridecadiennitril (Isomerengemisch) | 2,0 |
| Orangenöl (brasilian.) | 90,0 |
| Cabreuvaöl | 20,0 |
| Citronellol | 50,0 |
| Benzylacetat | 150,0 |
| Ylang Ylangöl | 10,0 |
| Benzylsalicylat | 20,0 |
| Iraldein | 80,0 |
| 4-tert-Butylcyclohexylacetat | 60,0 |
| Patchouliöl | 20,0 |
| Cyclopentadecanolid | 30,0 |
| Moschus Keton | 30,0 |
| Dipropylenglykol | 136,0 |
| | 1000,0 |

Der Einsatz von 3,11-Tridecadiennitril (Isomerengemisch) bewirkt auch in dieser Komposition eine größere Duftfülle und eine größere Natürlichkeit.

**Patentansprüche**

**1.** Alkadiennitrile der Formel

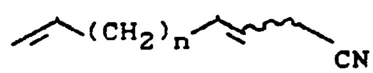

(I)

in der n eine ganze Zahl von 5 bis 9 ist.

**2.** Verfahren zur Herstellung von Alkadiennitrilen der allgemeinen Formel

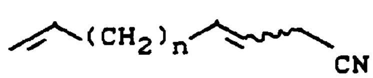

(I)

in der n eine ganze Zahl von 5 bis 9, dadurch gekennzeichnet, daß man Alkenale der Formel

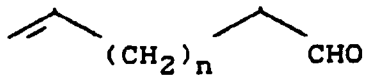

(II),

in der n die unter Formel (I) angegebene Bedeutung hat, mit Cyanessigsäure in Gegenwart von N,N-Dimethylbenzylamin kondensiert.

3. Verwendung der Alkadiennitrile nach Anspruch 1 als Riechstoffe.

## Claims

1. Alkadienenitriles of the formula

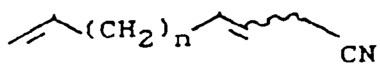

(I)

in which n is an integer from 5 to 9.

2. Process for the preparation of alkadienenitriles of the general formula

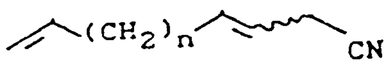

(I)

in which n is an integer from 5 to 9, characterized in that alkenals of the formula

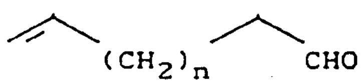

(II)

in which n has the meaning given under formula (I), are subjected to a condensation reaction with cyanoacetic acid in the presence of N,N-dimethylbenzylamine.

3. Use of the alkadienenitriles according to Claim 1 as odoriferous substances.

## Revendications

1. Alcadiènenitriles de formule :

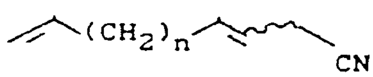

(I)

dans laquelle n représente un nombre entier compris entre 5 et 9.

2. Procédé de préparation d'alcadiènenitriles de formule générale :

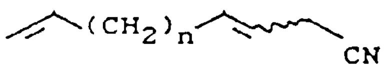

(I)

dans laquelle n représente un nombre entier compris entre 5 et 9, caractérisé en ce qu'on condense des aldéhydes éthyléniques de formule :

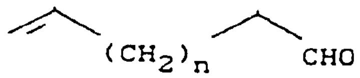

(II),

dans laquelle n a la signification donnée en dessous de la formule (I), avec de l'acide cyanacétique, en présence de N,N-diméthylbenzylamine.

3. Utilisation des alcadiènenitriles selon la revendication 1 en tant que parfums.